(19) Europäisches Patentamt
European Patent Office
Office européen des brevets

(11) Veröffentlichungsnummer: **0 001 207**
**B1**

(12) # EUROPÄISCHE PATENTSCHRIFT

(21) Anmeldenummer: **78100623.4**

(22) Anmeldetag: **08.08.78**

(51) Int. Cl.³: **C 07 D 498/10,**
C 07 D 498/20,
C 08 K 5/35, //
(C 07 D 498/10, 263/00,
221/00), (C 07 D 498/20,
263/00, 221/00)

(54) **Harnstoffderivate, ihre Herstellung und Verwendung als Lichtschutzmittel**

(30) Priorität: **25.08.77 DE 2738340**

(43) Veröffentlichungstag der Anmeldung:
**04.04.79 Patentblatt 79/07**

(45) Bekanntmachung des Hinweises auf die Patenterteilung:
**01.10.80 Patentblatt 80/20**

(84) Benannte Vertragsstaaten:
**BE CH DE FR GB NL**

(56) Entgegenhaltungen:
**DE - A - 2 118 339**
**DE - A - 2 265 271**
**DE - A - 2 500 313**
**DE - A - 2 634 957**
**US 3 941 744**

(73) Patentinhaber: **HOECHST AKTIENGESELLSCHAFT**
**Postfach 80 03 20**
**D - 6230 Frankfurt/Main 80 (DE)**

(72) Erfinder: **Wiezer, Hartmut, Dr.**
**Hans-Fischer-Strasse 6**
**D - 8906 Gersthofen (DE)**
**Pfahler, Gerhard, Dr.**
**Karlsbader Strasse 27**
**D - 8900 Augsburg (DE)**
**Mayer, Norbert, Dr.**
**Ziegelgrundweg 17**
**D - 8901 Gablingen (DE)**
**Knorr, Harald, Dr.**
**Hans-Fischer-Strasse 6**
**D - 8906 Gersthofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Courier Press, Leamington Spa, England.

# Harnstoffderivate, ihre Herstellung und Verwendung als Lichtschutzmittel

Die Erfindung betrifft neue Harnstoffderivate, deren Herstellung und ihre Verwendung als Stabilisatoren für synthetische Polymere.

Die neuen Harnstoffderivate sind Verbindungen der Formel (I)

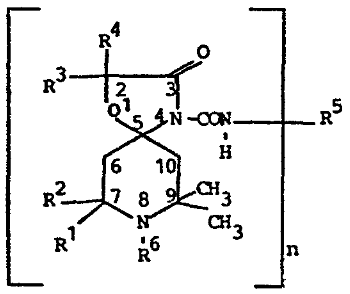

I

in der

n für die Zahl 1 oder 2 steht,

$R^1$ und $R^2$ gleiche oder verschiedene geradkettige oder verzweigte Alkylreste mit 1 bis 12, vorzugsweise 1 bis 6 C-Atomen und insbesondere Methylgruppen sind, oder $R^1$ und $R^2$ zusammen mit dem Kohlenstoffatom, an das sie gebunden sind, einen gegebenenfalls methylsubstituierten Cyclopentan- oder Cyclohexanring oder einen 2.2.6.6-Tetramethylpiperidinring, dessen Kohlenstoff 4 mit dem Kohlenstoffatom 7 des Spirodecansystems identisch ist, vorzugsweise letztgenannten, bilden,

$R^3$ für Wasserstoff, Alkyl- oder Isoalkylreste mit 1 bis 30, vorzugsweise 1 bis 10 und insbesondere 1 bis 6 C-Atomen oder einen Aralkylrest mit 7 bis 10 C-Atomen, wobei die aliphatische Kette 1 bis 4 C-Atome besitzt, steht,

$R^4$ Wasserstoff, eine Alkylgruppe mit 1 bis 30, vorzugsweise 1 bis 17 und insbesondere 1 bis 11 C-Atomen, ein gegebenenfalls durch ein Halogenatom—bevorzugt Chlor—oder einen Alkylrest mit 1 bis 4 C-Atomen substituierter Arylrest mit 6 oder 10 C-Atomen oder ein Aralkylrest mit 7 bis 10 C-Atomen, wobei 1 bis 4 C-Atome zur aliphatischen Kette gehören, ist, oder auch

$R^3$ und $R^4$ mit dem sie bindenden Kohlenstoffatom einen Cycloalkanring mit 4 bis 20, vorzugsweise 5 bis 12 und insbesondere 5 bis 7 C-Atomen, wobei der Cycloalkanring durch $C_1$- bis $C_4$-Alkylreste substituiert sein kann, bilden.

$R^5$ bei n = 1 eine Alkylgruppe mit 1 bis 20, vorzugsweise 4 bis 18 C-Atomen, eine Alkenylgruppe mit 3 bis 18 C-Atomen, eine gegebenenfalls durch einen $C_1$- bis $C_4$-Alkylrest substituierten Cycloalkylrest mit 5 bis 12, vorzugsweise 5 bis 7 und insbesondere 6 C-Atomen, einen gegebenenfalls durch ein Chloratom oder einen Alkylrest mit 1 bis 18, vorzugsweise 1 bis 4 C-Atomen substituierten Arylrest mit 6 oder 10 C-Atomen oder einen Aralkylrest mit 7 bis 18, vorzugsweise 7 bis 10 C-Atomen, wobei der Arylring 6 C-Atomen enthält, bedeutet, während

$R^5$ bei n = 2 geradkettiges oder verzweigtes Alkylen mit 2 bis 20, vorzugsweise 2 bis 12 und insbesondere 2 bis 6 C-Atomen, gegebenenfalls durch $C_1$-bis $C_4$-Alkyl substituiertes Arylen mit 6 oder 10 C-Atomen oder Aralkylen mit 7 bis 18 C-Atomen ist, und

$R^6$ Wasserstoff, Sauerstoff, Hydroxyl oder einen $C_1$- bis $C_4$-Alkylrest, vorzugsweise Wasserstoff und Methyl, insbesondere Wasserstoff, bedeutet.

Da das Stickstoffatom 8 basische Eigenschaften aufweist, wenn es mit H oder Alkyl substituiert ist, können die Verbindungen in diesen Fällen auch in Form von Salzen mit anorganischen oder organischen Säuren vorliegen.

Beispiele für die Reste $R^1$ und $R^2$ sind z.B. Methyl, Äthyl, i-Butyl, für Reste, in denen $R^1$ und $R^2$ zusammen mit dem Kohlenstoffatom, an das sie gebunden sind, einen Ring bilden z.B. Cyclopentyl, Cyclohexyl oder 2.2.6.6-Tetramethylpiperidyl.

Beispiele für $R^3$ sind Wasserstoff, Methyl, Äthyl, Propyl, Butyl, Pentyl, Hexyl, Heptyl, Benzyl, für $R^4$ Wasserstoff, Methyl, Äthyl, Propyl, Isopropyl, Butyl, Isobutyl, Pentyl, Isopentyl, Hexyl, Isohexyl, Heptyl, Isoheptyl, Octyl, Isooctyl, Nonyl, Isononyl, Decyl, Isodecyl, Undecyl, Dodecyl, Tridecyl, Benzyl, Phenyl, Chlorphenyl, Phenyläthyl.

Beispiele für solche Fälle, in denen $R^3$ und $R^4$ zusammen mit dem Ring-C-Atom 2, an das sie gebunden sind, einen Cycloalkylring bilden, sind Cyclopentyl, Cyclohexyl, Methylcyclohexyl, Cycloheptyl und Cyclododecyl.

Beispiele für Reste $R^5$ bei Verbindungen mit n = 1 sind die Reste von Monoisocyanaten, z.B. Methyl, Äthyl, Propyl, Butyl, Isobutyl, Octadecyl, Cyclohexyl, Phenyl, 3-Chlorphenyl, 4-Chlorphenyl, 3-Methylphenyl, 4-Methylphenyl, 1-Naphthyl.

Bei n = 2 bedeutet $R^5$ den Rest eines Diisocyanates, z.B. Athylen, Hexamethylen, Diphenylenmethan, 4-Methyl-m-phenylen.

Beispiele für $R^6$ sind Wasserstoff und Niedrigalkyl, insbesondere Methyl.

Beispiele für mögliche Salze der Verbindungen der Formel I sind Salze mit anorganischen Säuren wie Phosphate, Phosphite, Chloride, Sulfate und Salze mit organischen Mono- und Polycarbonsäuren wie Acetate, Laurate, Stearate, Succinate, Sebacate, Maleate, Citrate, Tartrate, Oxalate, Benzoate, Sulfonate, Phosphonate etc.

Von den unter die allgemeine Formel I fallenden Verbindungen seien folgende beispielhaft aufgeführt:

2.2.7.7.9.9-Hexamethyl-4-α-Naphthyl-carbamoyl-1-oxa-3-oxo-4,8-diaza-spiro-[4,5]-decan;
2.2.7.7.9.9-Hexamethyl-4-cyclohexyl-carbamoyl-1-oxa-3-oxo-4,8-diaza-spiro-[4,5]-decan;
2.2.7.7.9.9-Hexamethyl-4-phenyl-carbamoyl-1-oxa-3-oxo-4,8-diaza-spiro-[4,5]-decan;
2.2.7.7.9.9-Hexamethyl-4-octadecyl-carbamoyl-1-oxa-3-oxo-4,8-diaza-spiro-[4,5]-decan;
Diphenylen - methan - 4',4'' - bis - (4 - carbamoyl - 2.2.7.7.9.9 - hexamethyl - 1 - oxa - 3 - oxo - 4,8-diaza - spiro - [4,5] - decan);
n-Hecan-1'.6'-bis-(4-carbamoyl-2.2.7.7.9.9-hexamethyl-1-oxa-3-oxo-4,8-diaza-spiro-[4,5]-decan);
n - Hexan - 1' - 6' - bis - (4 - carbamoyl - 2.7.7.9.9 - pentamethyl - 2 - propyl - 1 - oxa - 3 - oxo - 4,4-diaza - spiro - [4,5] - decan);
2.7.7.9.9-Pentamethyl-2-iso-butyl-4-phenyl-carbamoyl-1-oxa-3-oxo-4,8-diaza-spiro-[4,5]-decan;
Diphenylen-methan-4'4''-bis-(4-carbamoyl-2.7.7.9.9-pentamethyl-2-pentyl-1-oxa-3 - oxo - 4,8 - diaza-spiro-[4,5]-decan);
n-Hexan-1'.6'-bis-(4-carbamoyl-2.7.7.9.9-pentamethyl-2-hexyl-1-oxa-3-oxo-4,8 - diaza - spiro - [4,5]-decan);
n-Hexan-1'.6'-bis-(4-carbamoyl-2.2-diäthyl-7.7.9.9-tetramethyl-1-oxa-3-oxo-4,8-diaza - spiro - [4,5]-decan);
2.2-Dipropyl-7.7.9.9-tetramethyl-4-octadecyl-carbamoyl-1-oxa-3-oxo-4,5-diaza-spiro-[4,5]-decan;
2-Propyl-7.7.9.9-tetramethyl-4-propyl-carbamoyl-1-oxa-3-oxo-4,8-diaza-spiro-[4,5]-decan;
2-iso-Butyl-7.7.9.9-tetramethyl-4-cyclohexyl-carbamoyl-1-oxa-3-oxo-4,8-diaza-spiro-[4,5]-decan;
n-Hexan-1'.6'-bis-(4-carbamoyl-2-iso-butyl-7.7.9.9-tetramethyl-1-oxa-3-oxo-4,8 - diaza - spiro - [4,5]-decan);
n-Hexan-1'.6'-bis-(4-carbamoyl-2-iso-pentyl-7.7.9.9-tetramethyl-1-oxa-3-oxo-4,8-diaza - spiro - [4,5]-decan);

Die neuen Verbindungen der allgemeinen Formel (I) werden durch Umsetzen von Isocyanaten bzw. Diisocyanaten (II) mit Piperidinen der allgemeinen Formel (III), die nach den Angaben der DE-OS 2 634 957 zugänglich sind, erhalten. Den Reaktionsablauf gibt die nachfolgende Gleichung wieder.

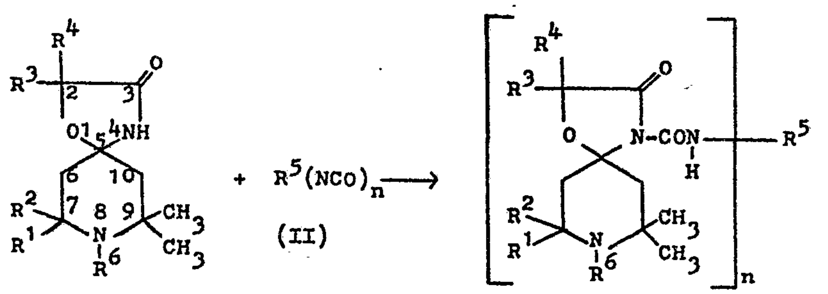

(III) (I)

Die Reste $R^1$, $R^2$, $R^3$, $R^4$, $R^5$ und $R^6$ haben die früher angegebene Bedeutung, n ist 1 oder 2 entsprechend der Funktionellität des Isocyanats. Besonders überraschend ist bei dieser Reaktion die Tatsache, daß auch solche Verbindungen nach Formel (III), in denen $R^6$ die Bedeutung von Wasserstoff hat, in der angegebenen Weise mit Isocyanaten reagieren und nicht am Aminstickstoff in der Stellung 8.

Bei der Umsetzung geht man in der Weise vor, daß man das Piperidinderivat in einem inerten organischen Lösungsmittel vorlegt, eine katalytische Menge einer starken Base wie z.B. 1,4-Diazabicyclooctan zusetzt, die äquivalente Menge des Isocyanats, gegebenenfalls in demselben Lösungsmittel gelöst, zutropft und bei 20 bis 180, vorzugsweise 20 bis 140 und insbesondere 50 bis 120°C reagieren läßt. Die Reaktionszeit beträgt 1 bis 30, vorzugsweise 4 bis 20 Stunden.

Geeignete inerte organische Lösungsmittel sind z.B. Heptan, aliphatische Kohlenwasserstoffschnitte mit Siedebereichen bis 180°C, Toluol, Xylol, Diäthyläther, Dioxan Halogenkohlenwasserstoffe wie Chloroform, Tetrachlorkohlenstoff, Chlorbenzol, bevorzugt ist Toluol.

Schwer lösliche Reaktionsprodukte werden durch Filtration abgetrennt, gelöst bleibende reinigt man nach dem Abdampfen des Lösungsmittels durch Umkristallisieren.

Die neuen Harnstoffderivate eignen sich hervorragend zum Stabilisieren von synthetischen Polymeren gegen die zersetzenden Einwirkungen von Licht, wobei unter "synthetischen Polymeren" in

diesem Zusammenhang verstanden werden: Halogenfreie und halogenhaltige Homo- und Copolymere, im einzelnen Homopolymerisate von Olefinen, Dienen und Styrol, wie z.B. Polyäthylen niedriger und hoher Dichte, Polypropylen, Polystyrol, Polybutadien und Polyisopren, Copolymere von Olefinen, Dienen und Styrol miteinander oder mit anderen olefinisch ungesättigten Monomeren, wie Äthylen-Propylen-Copolymere, Äthylen-Buten-Copolymere, Styrol-Butadien-Copolymere, Äthylen-Vinylacetat-Copolymere und Acrylnitril-Butadien-Styrol-Copolymere, Homopolymere von Vinylchlorid und Vinylidenchlorid und Copolymere dieser Monomeren untereinander und mit anderen olefinisch ungesättigten Monomeren. Des weiterin sollen auch Polyurethane, Polyacetale, Polyester, Polyamide, Polyacrylate und Epoxyharze eingeschlossen sein. Bevorzugt sind Poly-$\alpha$-Olefine wie Polyäthylene und insbesondere Polypropylene, sowie die Polymeren des Vinylchlorids.

Es war überraschend und nicht vorhersehbar, daß die erfindungsgemäßen Produkte bekannten, in den DE-OSS 2 500 313, 1 770 689 und 1 769 646 beschriebenen Verbindungen mit ähnlichen Strukturmerkmalen, d.h. Harnstoffen bzw. Spirodecanen mit ähnlichem Grundgerüst in ihrer UV-stabilisierenden Wirksamkeit merklich überlegen sind, es war vielmehr anzunehmen, daß aufgrund der an sich unwesentlich erscheinenden Veränderungen hinsichtlich der Struktur die Wirksamkeiten in derselben Größenordnung liegen oder sogar schlechter sein sollten, weil bei den neuen Verbindungen wegen ihrer Polarität eine weniger gute Verträglichkeit, besonders mit unpolaren Polymeren wie z.B. Polyolefinen, zu erwarten gewesen wäre.

Die neuen stabilisierenden Verbindungen werden nach allgemein üblichen Methoden in die Polymermassen eingearbeitet. Alternativ kann man auch eine Lösung, Suspension oder Emulsion des Stabilisators mit dem Polymeren direkt, oder mit einer Lösung, Suspension oder Emulsion desselben vermischen, und das Lösungsmittel anschließend entfernen.

Die erfindungsgemäßen Stabilisatoren sind für sich allein oder in Gemisch mit einem oder mehreren der bei der Kunststoffverarbeitung üblichen Stabilisatoren, wie z.B. Antioxidantien auf Phenol- und Sulfidbasis. UV-Absorbern und Lichtschutzmitteln, Phosphitstabilisatoren, Metallverbindungen, Epoxystabilisatoren und mehrwertigen Alkoholen einsetzbar. In den zu stabilisierenden Kunststoffmassen können ferner Flammschutzmittel und Pigmente, Farbstoffe, Antistatika und Füllstoffe wie z.B. Glasfasern, anwesend sein.

Beispiele für geeignete Antioxidantien sind solche vom Typ der sterisch gehinderten Phenole wie 2,6-Di-t.-butyl-p-kresol, 2,6-Di-octadecyl-p-kresol, 4,4′-Butyliden-bis-(2,6-di-t.-butyl-phenol), 4,4′-Thio-bis-(2-t.-butyl-5-methylphenol), phenolische Triazinverbindungen, Thiodipropionsäureester von Fettalkoholen, Dioctadecylsulfid und -disulfid.

Zu den UV-Absorbern und Lichtschutzmitteln gehören z.B. 2-(2′-Hydroxyphenyl)-benztriazole wie 2-(2′-Hydroxy-5′-methylphenyl)-benztriazol, 2-Hydroxybenzophenone wie 2-Hydroxy-4-octoxy-benzophenon, Stabilisatoren aus der Gruppe der Salizylate wie Octylphenylsalizylat, Nickelchelate, Oxalsäurediamide und sterisch gehinderte Piperidinverbindungen.

Als Phosphite sind Trisnonylphenylphosphit, Trislaurylphosphit oder auch Ester des Pentaerythritphosphits zu nennen.

Unter als Stabilisatoren bekannten Metallverbindungen werden in diesem Zusammenhang verstanden: Calcium-, Barium-, Strontium-, Zink-, Cadmium-, Magnesium-, Aluminium und Bleiseifen aliphatischer Carbonsäuren oder Oxycarbonsäuren mit etwa 12 bis 32 C-Atomen, Salze der genannten Metalle mit aromatischen Carbonsäuren wie Benzoate oder Salizylate sowie (Alkyl)-Phenolate dieser Metalle, ferner Organozinnverbindungen, wie z.B. Dialkylzinnthioglykolate und Carboxylate.

Bekannte Epoxystabilisatoren sind z.B. epoxidierte höhere Fettsäuren wie epoxidiertes Sojabohnenöl, Tallöl, Leinöl oder epoxidiertes Butyloleat sowie Epoxide langkettiger Olefine.

Mehrwertige Alkohole können beispielsweise Pentaerythrit, Trimethylolpropan, Sorbit oder Mannit sein, d.h. bevorzugt Alkohole mit 5 oder 6 C-Atomen und 3 bis 6 OH-Gruppen.

Eine wirksame Stabilisatorkombination für Poly-$\alpha$-Olefine, wie z.B. Hoch-, Mittel- und Niederdruckpolymerisate von $C_2$- bis $C_4$-$\alpha$-Olefinen, insbesondere Polyäthylen und Polypropylen oder von Copolymerisaten derartiger $\alpha$-Olefine besteht, bezogen auf 100 Gewichtsteile Polymer, beispielsweise aus 0,01 bis 5 Gewichtsteilen einer der erfindungsgemäß zu verwendenden Verbindungen, 0,5 bis 5 Gewichtsteilen eines phenolischen Stabilisators, gegebenenfalls 0,01 bis 5 Gewichtsteilen eines schwefelhaltigen Costabilisators sowie gegebenenfalls 0,01 bis 3 Gewichtsteilen einer basischen oder neutralen Metallseife, wie z.B. Calciumstearat oder Zinkstearat sowie gegebenenfalls 0,1 bis 5 Gewichtsteilen eines Phosphits und gegebenenfalls 0,01 bis 5 Gewichtsteilen eines bekannten UV-Stabilisators aus der Gruppe der Alkoxyhydroxybenzophenone, Hydroxyphenylbenztriazole, Benzylidenmalonsäuremononitrilseester oder der sogenannten Quencher, wie z.B. Nickelchelate.

Die folgenden Beispiele dienen der näheren Erläuterung der Erfindung. Die Strukturen der Verbindungen wurden kernresonanzspektroskopisch ermittelt.

### Beispiel 1

**2.2.7.7.9.9-Hexamethyl-4-$\alpha$-Naphthyl-carbamoyl-1-oxa-3-oxo-4,8-diaza-spiro-[4,5]-decan**

12,0 g (0,05 Mol) 2.2.7.7.9.9-Hexamethyl-1-oxa-4,8-diaza-spiro-[4,5]-decan, 8,5 g (0,05 Mol) Naphthyl-1-isocyanat, 0,1 g 1.4-Diazabicyclooctan und 100 ml absolutes Toluol werden

unter Rühren 15 Stunden rückfließend gekocht. Anschließend wird das Toluol abdestilliert und der Rückstand aus Heptan umkristallisiert. Fp. 158°C.

Beispiele 2 bis 16

Entsprechend den Angaben des Beispieles 1 wurden hergestellt:

| Beispiel Nr. | Verbindung / Fp. / Ausgangsmaterial |
|---|---|
| 2 | 2.2.7.7.9.9 - Hexamethyl - 4 - cyclohexyl - carbamoyl - 1 - oxa - 3 - oxo - 4,8 - diaza-spiro - [4,5] - decan (Fp. 148°C) aus 2.2.7.7.9.9 - Hexamethyl - 1 - oxa - 3 - oxo - 4,8-diaza - spiro - [4,5] - decan und $C_6H_{11}NCO$ |
| 3 | 2.2.7.7.9.9 - Hexamethyl - 4 - phenyl - carbamoyl - 1 - oxa - 3 - oxo - 4,8 - diaza - spiro-[4,5] - decan (Fp. 174°C) aus 2.2.7.7.9.9 - Hexamethyl - 1 - oxa - 3 - oxo - 4,8 - diaza-spiro - [4,5] - decan und $C_6H_5NCO$ |
| 4 | 2.2.7.7.9.9 - Hexamethyl - 4 - octadecyl - carbamoyl - 1 - oxa - 3 - oxo - 4,8 - diaza - spiro-[4,5] - decan (Fp. 65°C) aus 2.2.7.7.9.9 - Hexamethyl - 1 - oxa - 3 - oxo - 4,8 - diaza-spiro - [4,5] - decan und $C_{18}H_{37}NCO$ |
| 5 | Diphenylen - methan - 4'.4'' - bis - (4 - carbamoyl - 2.2.7.7.9.9 - hexamethyl - 1 - oxa-3 - oxo - 4,8 - diaza - spiro - [4,5] - decan (Fp. 287°C) aus 2.2.7.7.9.9 - Hexamethyl-1 - oxa - 3 - oxo - 4,8 - diaza - spiro - [4,5] - decan und $CH_2(C_6H_4NCO)_2$ |
| 6 | n - Hexan - 1'.6' - bis - (4 - carbamoyl - 2.2.7.7.9.9 - hexamethyl - 1 - oxa - 3 - oxo-4,8 - diaza - spiro - [4,5] - decan (183—186°C) aus 2.2.7.7.9.9 - Hexamethyl - 1 - oxan-3 - oxo - 4,8 - diaza - spiro - [4,5] - decan und $(CH_2)_6(NCO)_2$ |
| 7 | 2.7.7.9.9 - Pentamethyl - 2 - iso - butyl - 4 - phenyl - carbamoyl - 1 - oxa - 3 - oxo-4,8 - diaza - spiro - [4,5] - decan (86—88°C) aus 2.7.7.9.9 - Pentamethyl - 2 - iso - butyl-1 - oxa - 3 - oxo - 4,8 - diaza - spiro - [4,5] - decan und $C_6H_5NCO$ |
| 8 | Diphenylen - methan - 4'.4'' - bis - (4 - carbamoyl - 2.7.7.9.9 - pentamethyl - 2 - pentyl-1 - oxa - 3 - oxo - 4,8 - diaza - spiro - [4,5] - decan (298°C aus 2.7.7.9.9 - Pentamethyl - 2-pentyl - 1 - oxa - 3 - oxo - 4,8 - diaza - spiro - [4,5] - decan und $CH_2(C_6H_4NCO)_2$ |
| 9 | n - Hexan - 1'.6' - bis - (4 - carbamoyl - 2.2.diäthyl - 7.7.9.9 - tetramethyl - 1 - oxa - 3-oxo - 4,8 - diaza - spiro - [4,5] - decan) (117°C) aus 2.2 - Diäthyl - 7.7.9.9 - tetramethyl-1 - oxa - 3 - oxo - 4,8 - diaza - spiro - [4,5] - decan und $(CH_2)_6(NCO)_2$ |
| 10 | n - Hexan - 1'.6' - bis - (4 - carbamoyl - 2.7.7.9.9 - penta - methyl - 2 - hexyl - 1 - oxa-3 - oxo - 4,8 - diaza - spiro - [4,5] - decan (116—118°C) aus 2.7.7.9.9 - Pentamethyl-2 - hexyl - 1 - oxa - 3 - oxo - 4,8 - diaza - spiro - [4,5] - decan und $(CH_2)_6(NCO)_2$ |
| 11 | 2.2 - Dipropyl - 7.7.9.9 - tetramethyl - 4 - octadecyl - carbamoyl - 1 - oxa - 3 - oxo-4,8 - diaza - spiro - [4,5] - decan (85—90°C) aus 2.2 - dipropyl - 7.7.9.9 - tetramethyl-1 - oxa - 3 - oxo - 4,8 - diaza - spiro - [4,5] - decan und $C_{18}H_{37}NCO$ |
| 12 | 2 - Propyl - 7.7.9.9 - tetramethyl - 4 - propyl - carbamoyl - 1 - oxa - 3 - oxo - 4,8 - diaza-spiro - [4,5] - decan (60°C aus 2 - Propyl - 7.7.9.9 - tetramethyl - 1 - oxa - 3 - oxo-4,8 - diaza - spiro - [4,5] - decan und $C_3H_7NCO$ |
| 13 | 2 - iso - Butyl - 7.7.9.9 - tetramethyl - 4 - cyclohexyl - carbamoyl - 1 - oxa - 3 - oxo - 4,8-diaza - spiro - [4,5] - decan (150°C) aus 2 - iso - Butyl - 7.7.9.9 - tetramethyl - 1 - oxa-3 - oxo - 4,8 - diaza - spiro - [4,5] - decan und $C_6H_{11}NCO$ |
| 14 | n - Hexan - 1'.6' - bis - (4 - carbamoyl - 2.7.7.9.9 - pentamethyl - 2 - propyl - 1 - oxa - 3-oxo - 4,8 - diaza - spiro - [4,5] - decan) (80—85°C) aus 2.7.7.9.9 - Pentamethyl - 2-propyl - 1 - oxa - 3 - oxo - 4,8 - diaza - spiro - [4,5] - decan und $(CH_2)_6(NCO)_2$ |
| 15 | n - Hexan - 1'.6' - bis - (4 - carbamoyl - 2 - iso - butyl - 7.7.9.9 - tetramethyl - 1 - oxa - 3-oxo - 4,8 - diaza - spiro - [4,5] - decan) (115—117°C) aus 2 - iso - Butyl - 7.7.9.9 - tetramethyl - 1 - oxa - 3 - oxo - 4,8 - diaza - spiro - [4,5] - decan und $(CH_2)_6(NCO)_2$ |

| Beispiel Nr. | Verbindung / Fp. / Ausgangsmaterial |
|---|---|
| 16 | n - Hexan - 1'.6' - bis - (4 - carbamoyl - 2 - iso - pentyl - 7.7.9.9 - tetramethyl - 1 - oxa-3 - oxo - 4,8 - diaza - spiro - [4,5] - decan) (109°C) aus 2 - iso - Pentyl - 7.7.9.9 - tetramethyl - 1 - oxa - 3 - oxo - 4,8 - diaza - spiro - [4,5] - decan und $(CH_2)_6(NCO)_2$. |

Beispiel 17

Dieses Beispiel zeigt die lichtstabilisierende Wirkung der erfindungsgemäßen Verbindungen beim Einsatz in einem Poly-$\alpha$-Olefin.

100 Gewichtsteile Polypropylen mit einem Schmelzindex $i_5$ von ca. 6 g/10 min. (bestimmt nach ASTM D 1238—62 T) und einer Dichte von 0,96 wurden mit
0,1 Gew.-Teilen Pentaerythrityl - tetrakis - [3 - (3,5 - di -tert. - butyl - 4 - hydroxyphenyl) - propionat],
0,2 Gew.-Teilen Calciumstearat und
0,1.-Gewichtsteilen des zu prüfenden erfindungsgemäßen Stabilisators vermischt.

Um eine möglichst gleichmäßige Verteilung auf dem Polymerkorn zu erreichen, wurden die Stabilisatoren in einem Lösungsmittel gelöst, und die Lösung wurde unter Rühren in das Polypropylenpulver eingetropft, wobei durch gleichzeitige Bestrahlung mit einer IR-Lampe der größte Teil des Lösemittels wieder abdampfte. Nach ca. 20 min. wurde das Calciumstearat hinzugegeben und noch weitere 10 min. gemischt. Lösemittelreste wurden durch Trocknen bei 50°C/120 min. im Trockenschrank entfernt.

Daraus wurden auf einer Windsor-Spritzgußmaschine der Type SP 50 bei 250°C 60 x 60 x 1 mm-Platten verspritzt. Aus diesen Platten wurden Prüfkörper nach DIN 53 455 Form 3. verkleinert im Maßstab 1:3, ausgestanzt. Die als Vergleichsmuster benötigen Prüfkörper wurden analog, jedoch unter Fortlassen des zu testenden Stabilisators (Versuch g) bzw. unter Verwendung bekannter Lichtstabilisatoren (Versuche e und f), hergestellt.

Die Prüfung der Lichtbeständigkeit wurde in einer Xenotest-450-Apparatur der Firma Original Hanau Quarzlampen GmbH mit der Filterkombination 6 IR + 1 UV gemäß DIN 53 387 "Kurzprüfung der Wetterbeständigkeit" geprüft. Während der Belichtungszeit betrug die Schwarztafeltemperatur 43°C + ± 1°C, die relative Luftfeuchtigkeit im Probenraum 70% ± 1%. Der Probenraum wurde alle 2 Stunden 5 min. lang mit Frischluft gespült. Die Reißdehnung wurde auf einer Zugprüfmaschine der Firma Instron bei einer Abzugsgeschwindigkeit von 5 cm/min. nach einer definierten Belichtungszeit ermittelt. Die Ergebnisse sind in der nachstehenden Tabelle zusammengestellt.

Der Stabilitätsfaktor ergibt sich aus dem Verhältnis der Bestrahlungszeit des stabilisierten zur Bestrahlungszeit des nicht stabilisierten Probekörpers, wobei jeweils so lange bestrahlt wurde, bis die Reißdehnung auf die Hälfte des Ausgangswertes gefallen ist.

Wie ersichtlich, ist die stabilisierende Wirkung besser als mit einem Benzophenon- bzw. einem Benzotriazol-Stabilisator. Sie ist auch besser als mit n-Hexan-1.6-bis-(1.4-carbamoyl-7.14-diaza-dispiro-[5.1.5.2]-pentadecan-15-on), dem Produkt nach Beispiel 3 der DT-OS 2 500 313, welches entsprechend dem dortigen Prüfbeispiel 11 einen Faktor von nur 3.1 besitzt.

| Versuch Nr. | Stabilisator nach Beispiel | Stabilitätsfaktor |
|---|---|---|
| a) | 4 | >5 |
| b) | 6 | >5 |
| c) | 10 | >5 |
| d) | 15 | >5 |
| e) | Benzophenon-Stabilisator[1)] | <2,5 |
| f) | Benzotriazol-Stabilisator[2)] | <2,5 |
| g) | Kontrolle (ohne Stabilisator) | 1 |

1) 2-hydroxy-4-n-octyloxybenzophenon
2) 2-Hydroxy-3'.5'-di-tert.-butylphenyl)-5-chlorbenzotriazol

**Patentansprüche**

1. Harnstoffderivate der allgemeinen Formel (I)

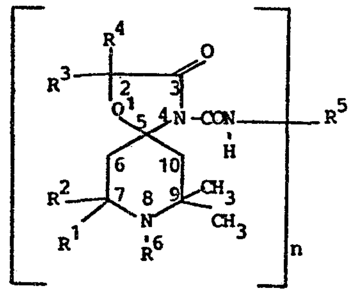

in der
n für die Zahl 1 oder 2 steht,
$R^1$ und $R^2$ gleiche oder verschiedene geradkettige oder verzweigte Alkylreste mit 1 bis 12 C-Atomen sind, oder $R^1$ und $R^2$ zusammen mit dem Kohlenstoffatom, an das sie gebunden sind, einen gegebenenfalls methylsubstituierten Cyclopentan- oder Cyclohexanring oder einen 2.2.6.6-Tetramethylpiperidinring, dessen Kohlenstoffatom 4 mit dem Kohlenstoffatom 7 des Spirodecansystems identisch ist, bilden,
$R^3$ für Wasserstoff, einen Alkyl- oder Isoalkylrest mit 1 bis 30 C-Atomen oder einen Aralkylrest mit 7 bis 10 C-Atomen, wobei die aliphatische Kette 1 bis 4 C-Atome besitzt, steht,
$R^4$ Wasserstoff, eine Alkylgruppe mit 1 bis 30 C-Atomen, ein gegebenenfalls durch ein Halogenatom oder einen Alkylrest mit 1 bis 4 C-Atomen substituierter Arylrest mit 6 oder 10 C-Atomen oder ein Aralkylrest mit 7 bis 10 C-Atomen, wobei 1 bis 4 C-Atome zur aliphatischen Kette gehören, ist, oder auch
$R^3$ und $R^4$ mit dem sie bindenden Kohlenstoffatom einen Cycloalkanring mit 4 bis 20 C-Atomen, wobei der Cycloalkanring durch $C_1$- bis $C_4$-Alkylreste substituiert sein kann, bilden,
$R^5$ bei $n = 1$ eine Alkylgruppe mit 1 bis 20, eine Alkenylgruppe mit 3 bis 18 C-Atomen, einen gegebenenfalls durch einen $C_1$- bis $C_4$-Alkylrest substituierten Cycloalkylrest mit 5 bis 12 C-Atomen, einen gegebenenfalls durch ein Chloratom oder einen Alkylrest mit 1 bis 18 C-Atomen substituierten Arylrest mit 6 bis 10 C-Atomen oder einen Aralkylrest mit 7 bis 18 C-Atomen, wobei der Arylring 6 C-Atome enthält, bedeutet, während
$R^5$ bei $n = 2$ geradkettiges oder verzweigtes Alkylen mit 2 bis 20 C-Atomen, gegebenenfalls durch $C_1$- bis $C_4$-Alkyl substituiertes Arylen mit 6 oder 10 C-Atomen oder Aralkylen mit 7 bis 18 C-Atomen ist, und
$R^6$ Wasserstoff, Sauerstoff, Hydroxyl oder einen $C_1$- bis $C_4$-Alkylrest bedeutet.

2. Verbindungen gemäß Anspruch 1, in denen $R^1$ und $R^2$ Methylgruppen sind und $R^6 = H$ ist.

3. Verfahren zur Herstellung der Verbindungen nach den Ansprüchen 1 und 2, dadurch gekennzeichnet, daß man in einem inerten organischen Lösungsmittel bei Temperaturen von 20 bis 180°C unter Zusatz katalytischer Mengen einer Base eine Verbindung der Formel (III)

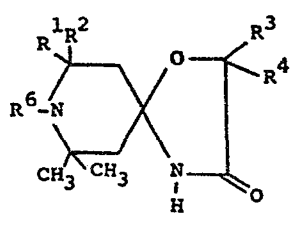

(III)

mit einem Isocyanat der Formel (II)

$$R^5(NCO)_n \qquad II$$

wobei n und die Reste $R^1$, $R^2$, $R^3$, $R^4$, $R^5$ und $R^6$ die in Anspruch 1 angegebene Bedeutung haben, umsetzt.

4. Verwendung der Verbindungen nach einem der Ansprüche 1 und 2, gegebenenfalls in Form des Salzes mit einer anorganischen oder organischen Säure, zum Stabilisieren von synthetischen Polymeren.

5. Verwendung nach Anspruch 4, dadurch gekennzeichnet, daß das Polymere ein Polyolefin ist.

6. Verwendung nach Anspruch 4, dadurch gekennzeichnet, daß das Polymere ein halogenhaltiges Polymeres ist.

7. Verfahren zum Stabilisieren von synthetischen Polymeren gegen den schädigenden Einfluß von Licht, dadurch gekennzeichnet, daß man den Polymeren gegebenenfalls neben bisher bekannten, stabilisierend wirkenden Stoffen, 0,01 bis 5 Gewichtsteile, bezogen auf Polymeres, einer Verbindung nach einem der Ansprüche 1 und 2 zusetzt, wobei diese Verbindung auch in Form eines Salzes mit einer anorganischen oder organischen Säure vorliegen kann.

8. Gegen UV-Zersetzung stabilisierte, synthetische Polymere, in denen 0,01 bis 5 Gewichtsteile, bezogen auf das Polymere, einer Verbindung nach einem der Ansprüche 1 und 2, gegebenenfalls in form des Salzes mit einer anorganischen oder organischen Säure, enthalten ist.

## Claims

1. Urea derivatives of the formula (I)

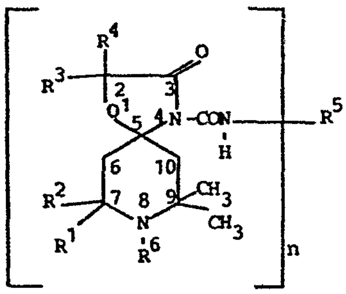

I

in which

n stands for 1 or 2,

$R^1$ and $R^2$, being identical or different, each are linear or branched alkyl radicals having from 1 to 12 carbon atoms, or $R^1$ and $R^2$ together with the carbon atom to which they are linked form an optionally methyl-substituted cyclopentane or cyclohexane ring or a 2,2,6,6-tetramethylpiperidine ring the carbon atom 4 of which is identical with the carbon atom 7 of the spirodecane system,

$R^3$ is hydrogen, an alkyl or isoalkyl radical having from 1 to 30 carbon atoms, or an aralkyl radical having from 7 to 10 carbon atoms, the aliphatic chain having from 1 to 4 carbon atoms;

$R^4$ is hydrogen, an alkyl group having from 1 to 30 carbon atoms, an aryl radical having 6 or 10 carbon atoms, optionally substituted by a halogen atom or an alkyl radical having from 1 to 4 carbon atoms, or an aralkyl radical having from 7 to 10 carbon atoms, the aliphatic chain containing from 1 to 4 of the latter carbon atoms; or

$R^3$ and $R^4$ together with the carbon atom linking them form a cycloalkane ring having from 4 to 20 carbon atoms, the cycloalkane ring optionally being substituted by $C_1$—$C_4$-alkyl groups;

$R^5$, when *n* is 1, is an alkyl group having from 1 to 20 carbon atoms, an alkenyl group having from 3 to 18 carbon atoms, a cycloalkyl radical having from 5 to 12 carbon atoms, optionally substituted by a $C_1$—$C_4$-alkyl radical, an aryl radical having 6 to 10 carbon atoms, optionally substituted by a chlorine atom or an alkyl radical having from 1 to 18 carbon atoms, or an aralkyl radical having from 7 to 18 carbon atoms, the aryl ring containing 6 carbon atoms;

$R^5$, when *n* is 2, is linear or branched alkylene having from 2 to 20 carbon atoms, arylene having 6 or 10 carbon atoms, optionally substituted by $C_1$—$C_4$-alkyl, or aralkylene having from 7 to 18 carbon atoms; and

$R^6$ is hydrogen, oxygen, hydroxyl or an alkyl radical having from 1 to 4 carbon atoms.

2. Compounds as claimed in claim 1, in which $R^1$ and $R^2$ are methyl groups and $R^6$ is H.

3. A process for the preparation of compounds as claimed in claims 1 and 2, which comprises reacting a compound of the formula (III)

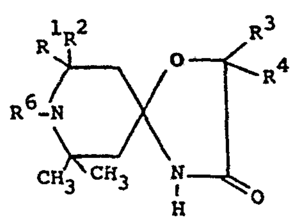

III

with an isocyanate of the formula (II)

$$R^5(NCO)_n \qquad II$$

in which formula n and $R^1$ to $R^6$ are as defined in claim 1, in an inert organic solvent at temperatures of from 20 to 180°C with addition of catalytic amounts of a base.

4. Use of the compounds as claimed in claims 1 and 2, optionally in the form of the salt of an inorganic or organic acid, for stabilizing synthetic polymers.

5. Use as claimed in claim 4, which comprises the polymer being a polyolefin.

6. Use as claimed in claim 4, which comprises the polymer being a halogen-containing polymer.

7. A process for stabilizing synthetic polymers against the damaging influence of light, which comprises adding to the polymers, optionally in addition to hitherto known stabilizing agents, from 0.01 to 5 parts by weight, relative to the polymer, of a compound as claimed in claims 1 and 2, optionally in the form of a salt of an inorganic or organic acid.

8. Synthetic polymers stabilized against UV decomposition, containing from 0.01 to 5 parts by weight, relative to the polymer, of a compound as claimed in claims 1 and 2, optionally in the form of a salt of an inorganic or organic acid.

**Revendications**

1. Dérivés de l'urée répondant à la formule générale (I)

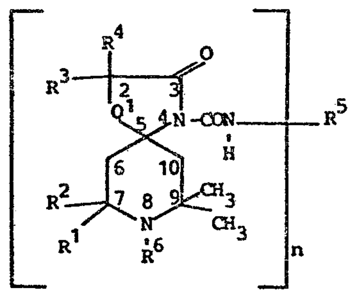

(I)

dans laquelle

n désigne le nombre 1 ou le nombre 2,

$R^1$ et $R^2$ représentent chacun, indépendamment l'un de l'autre, un radical alkyle, linéaire ou ramifié, contenant de 1 à 12 atomes de carbone, ou encore $R^1$ et $R^2$ forment ensemble et avec l'atome de carbone auquel ils sont liés un noyau de cyclopentane ou de cyclohexane éventuellement porteur d'un radical méthyle ou un noyau de tétraméthyl-2,2,6,6 pipéridine dont l'atome de carbone 4 coïncide avec l'atome de carbone 7 du système du spirodécane,

$R^3$ représente un atome d'hydrogène, un radical alkyle ou isoalkyle contenant de 1 à 30 atomes de carbone ou un radical aralkyle contenant de 7 à 10 atomes de carbone, la chaîne aliphatique en ayant de 1 à 4,

$R^4$ représente un atome d'hydrogène, un radical alkyle contenant de 1 à 30 atomes de carbone, un radical aryle à 6 ou à 10 atomes de carbone, éventuellement porteur d'un atome d'halogène ou d'un radical alkyle en $C_1$—$C_4$, ou un radical aralkyle contenant de 7 à 10 atomes de carbone dont 1 à 4 appartiennent à la chaîne aliphatique, ou encore

$R^3$ et $R^4$ forment avec l'atome de carbone qui les unit un noyau de cycloalcane contenant de 4 à 20 atomes de carbone, ce noyau de cycloalcane pouvant porter un radical alkyle en $C_1$—$C_4$,

$R^5$ représente, dans le cas où n est égal à 1, un radical alkyle contenant de 1 à 20 atomes de carbone, un radical alcényle contenant de 3 à 18 atomes de carbone, un radical cycloalkyle contenant de 5 à 12 atomes de carbone, éventuellement porteur d'un radical alkyle en $C_1$—$C_4$, un radical aryle ayant de 6 à 10 atomes de carbone, éventuellement porteur d'un atome de chlore ou d'un radical alkyle en $C_1$—$C_{18}$, ou un radical aralkyle contenant de 7 à 18 atomes de carbone et dont le noyau aryle en contient 6,

$R^5$ représente, dans le cas où n est égal à 2, un radical alkylène, linéaire ou ramifié, contenant de 2 à 20 atomes de carbone, un radical arylène à 6 ou à 10 atomes de carbone, éventuellement porteur d'un radical alkyle en $C_1$—$C_4$, ou un radical aralkylène contenant de 7 à 18 atomes de carbone, et

$R^6$ représente un atome d'hydrogène ou d'oxygène, un groupe hydroxy ou un radical alkyle en $C_1$—$C_4$.

2. Composés selon la revendication 1, dans lesquels $R^1$ et $R^2$ représentent chacun un radical méthyle et $R^6$ représente un atome d'hydrogène.

3. Procédé de préparation de composés selon l'une des revendications 1 et 2, procédé caractérisé en ce qu'on fait réagir un composé de formule III

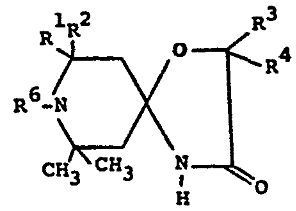

(III)

avec un isocyanate de formule II

$$R^5(NCO)_n \qquad (II)$$

(dans ces formules n, $R^1$, $R^2$, $R^3$, $R^4$, $R^5$ et $R^6$ ont les significations qui ont été données à la revendication 1), dans un solvant organique inerte, à des températures de 20 à 180°C, en présence de quantités catalytiques d'une base.

4. Utilisation de composés selon l'une des revendications 1 et 2, éventuellement sous la forme du sel avec un acide minéral ou organique, pour la stabilisation de polymères synthétiques.

5. Utilisation selon la revendication 4, caractérisée en ce que le polymère est une polyoléfine.

6. Utilisation selon la revendication 4, caractérisée en ce que le polymère est un polymère halogéné.

7. Procédé de stabilisation de polymères synthétiques contre l'action destructrice de la lumière, procédé caractérisé en ce qu'on ajoute aux polymères, éventuellement en plus de corps à action stabilisante connus jusqu'à présent, de 0,01 à 5 parties en poids, par rapport au polymère, d'un composé selon l'une des revendications 1 et 2, composé qui peut également se trouver sous la forme d'un sel avec un acide minéral ou organique.

8. Polymères synthétiques stabilisés contre la destruction par les rayons ultraviolets, caractérisés en ce qu'ils contiennent de 0,01 à 5 parties en poids, par rapport au polymère, d'un composé selon l'une des revendications 1 et 2, éventuellement sous la forme du sel avec un acide minéral ou organique.